Europäisches Patentamt

European Patent Office

Office européen des brevets

(11) Veröffentlichungsnummer: **0 240 457**
**A1**

## (12) EUROPÄISCHE PATENTANMELDUNG

(21) Anmeldenummer: 87730026.9

(22) Anmeldetag: 13.03.87

(51) Int. Cl.⁴: **A 61 B 17/16**

(30) Priorität: 15.03.86 DE 3609122

(43) Veröffentlichungstag der Anmeldung:
07.10.87 Patentblatt 87/41

(84) Benannte Vertragsstaaten:
CH DE ES FR GB IT LI

(71) Anmelder: **MECRON MEDIZINISCHE PRODUKTE GMBH**
**Nunsdorfer Ring 23-27**
**D-1000 Berlin 48 (DE)**

(72) Erfinder: **Ahrens, Uwe**
**Nürnberger Strasse 46**
**D-1000 Berlin 30 (DE)**

(74) Vertreter: **Christiansen, Henning, Dipl.-Ing.**
**CHRISTIANSEN & NINNEMANN Dietrich-Schäfer-Weg 21**
**D-1000 Berlin 41 (DE)**

(54) **Rotierendes Schneidinstrument für chirurgische Zwecke.**

(57) Ein rotierendes Schneidinstrument für chirurgische Zwecke mit Führungsspieß ermöglicht ein beschleunigtes Arbeiten dadurch, daß zwischen dem Führungsspieß (2) und der Wand eines für den Führungsspieß vorgesehenen Längskanals (3) des Schneidinstruments (I) ein Flüssigkeitskanal (I5) für die Zufuhr von Kühlmittel zum Schneidende (6) vorgesehen ist. Die Flüssigkeitszufuhr erfolgt über eine Muffe (7) und eine zugeordnete Queröffnung (I3). Der Führungsspieß (2) kann eine Längsabflachung (20) oder Rinne aufweisen, um trotz guten Sitzes im Längskanal (3) die Kühlmittelzufuhr zum Schneidende (6) zu gewährleisten.

## Beschreibung

### Rotierendes Schneidinstrument für chirurgische Zwecke

Die Erfindung betrifft ein rotierendes Schneidinstrument für chirurgische Zwecke mit Führungsspieß, für den im Schneidinstrument ein Längskanal vorgesehen ist.

Ein derartiges Schneidinstrument ist bekannt aus dem "MECRON Katalog, 1979/80, B. OSTEOSYNTHESE, B 33, Kata log-Nrn. 836 und 839". Es kann beispielsweise dazu dienen, einen kreiszylindrischen Kanal in einen Röhrenknochen zu bohren. Dabei wird zuerst ein Führungsspieß eingetrieben. Er dient dazu, den mit einem Längskanal, also hohlen Bohrer, beim Bohren zu führen, wenn dieser über den Führungsspieß geschoben, rotierend in die Markhöhle eindringt.

Da bei solchen Eingriffen stets Eile geboten ist, darf der Bohrvorgang nicht viel Zeit beanspruchen. Bei schnellem Bohren besteht aber die Gefahr, daß die entstehende Wärme nicht ausreichend abgeführt wird. Dann wären Knochen- oder Markschäden die Folge.

Der im kennzeichnenden Teil des Anspruchs I angegebenen Erfindung liegt die Aufgabe zugrunde, ein Schneidinstrument der eingangs genannten Gattungzu schaffen, das ein zügiges Arbeiten erlaubt.

Bei Bohrern ist es an sich bekannt, daß schneidende Ende durch dort aus dem Bohrer austretende Flüssigkeit zu kühlen. Dabei sind im Bohrer Längskanäle vorgesehen (DE-OS I75 2I 25 und DE-PS 23 3I 023), welche von der Kühlflüssigkeit durchflossen werden, die am schneidenden Bohrerende austritt. Die Flüssigkeitskanäle schwächen den Querschnitt eines solchen Bohrers. Deshalb ist es nicht möglich, die bekannten Anordnungen ohne weiteres auf ein rotierendes Schneidinstrument für chirurgische Zwecke mit einem Führungsspieß zu übertragen, denn dadurch, daß das rotierende Schneidinstrument in seinem inneren Längskanal bereits den Führungsspieß aufnehmen muß, ist der Querschnitt schon so geschwächt, daß für zusätzliche Flüssig keitskanäle kein Raum mehr ist. Es ist auch nicht möglich, die Flüssigkeitskanäle durch den Führungsspieß zu führen, denn dieser bedarf keiner Kühlung, und es ist auch nicht ausreichend, die am Schneidinstrument entstehende Wärme über den Umweg des Führungsspießes abzuführen.

Mit der Erfindung wird daher ein anderer Weg beschritten, der darin besteht, daß (zunächst wie bei einem der bekannten, flüssigkeitsgekühlten Bohrer) eine das Schneidinstrument umgreifende, abgedichtete und drehbar auf diesem gelagerte Muffe zur Kühlmittelzufuhr und eine zugeordnete Queröffnung im Schneidinstrument vorgesehen ist. Darüber hinaus ist nun aber im Schneidinstrument nicht zusätzlich zu dem für den Führungsspieß vorgesehenen Längskanal ein Flüssigkeitskanal vorgesehen, sondern der Flüssigkeitskanal wird durch einen Zwischenraum zwischen der Wand des Längskanals und dem Führungsspieß gebildet, wobei dieser Flüssigkeitskanal an demjenigen Ende des Schneidinstruments abgedichtet ist, aus welchem das stumpfe Ende des Führungsspießes (also gegenüber dem Schneidende des Schneidinstruments) aus dem Schneidinstrument herausragt.

Um eine gute Führung des Schneidinstruments durch den Führungsspieß zu erreichen, sollte dessen Durchmesser fast ebenso groß wie der Innendurchmesser des kreiszylindrischen Längskanals im Schneidinstrument sein; damit aber zwischen dem Führungsspieß und der Wand des Längskanals noch ein Flüssigkeitskanal mit ausreichend großem Querschnitt übrigbleibt, um genügend Kühlmittel hindurch zu lassen, kann der Führungsspieß von der Kreisform abweichende Ümfangsbereiche aufweisen. Beispielsweise kann der Führungsspieß bei ansonsten kreisförmigen Querschnitt an einer Längsseite abgeflacht sein oder eine Rille aufweisen.

Die zur Kühlmittelzufuhr dienende Muffe ist bevorzugt mit mindestens einem abgedichteten Wälzlager außen auf dem rotierenden Schneidinstrument gelagert.

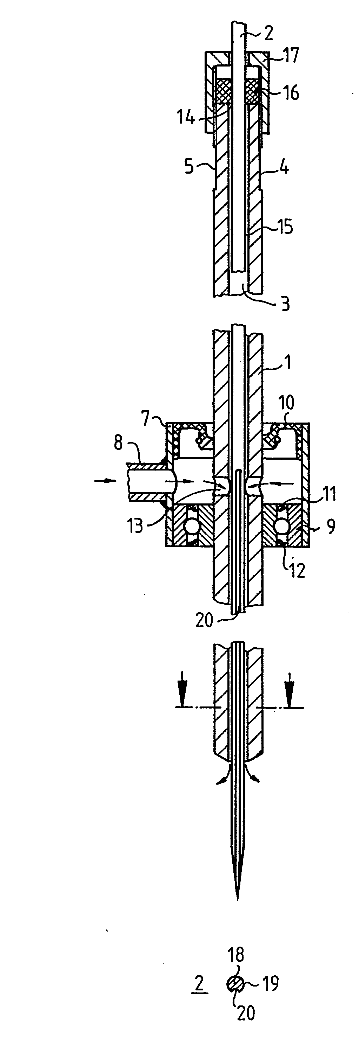

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Zeichnung näher dargestellt.

Im Schnitt ist als rotierendes Schneidinstrument I ein Bohrer mit Führungsspieß 2 in einem Längskanal 3 des Schneidinstruments I gezeigt. Beim Bohren steht der Führungsspieß 2 still, während das Schneidinstrument I über Nuten 4 und 5 von einem nicht dargestellten Bohrfutter angetrieben ist und mit seinem Schneidende 6 beispielsweise in eine Markhöhle eines Knochens eindringt.

Zur Abfuhr der beim Schneiden entstehenden Wärme wird nun flüssiges Kühlmittel zugeführt. Dazu ist eine das Schneidinstrument I abgedichtet und drehbar umfassende Muffe 7 mit einem Kühlmittelzuflußstutzen 8 vorgesehen. Zur leichtgängigen drehbaren Lagerung der Muffe 7 auf dem Schneidinstrument I ist dient ein Wälzlager 9. Die Abdichtung der Muffe 7 besorgen Dichtungsringe I0 bis I2.

Das flüssige Kühlmittel fließt in Pfeilrichtung durch die Muffe und von dieser durch eine zugeordnete Queröffnung I3 im Schneidinstrument I. Damit nun die einander widersprechenden Forderungen, daß einerseits der Querschnitt des Schneidinstruments I nicht weiter geschwächt wird und andererseits das Schneidinstrument I durch den Führungsspieß 2 möglichst gut und möglichst spielfrei geführt wird, wird der Zwischenraum zwischen dem Führungsspieß 2 und der Wand des Längskanals 3 als Flüssigkeitskanal genutzt. Durch ihn fließt das Kühlmittel und tritt am Schneidende 6 wieder aus dem Schneidinstrument I aus. Am gegenüberliegenden Ende I4 ist der Flüssigkeitskanal I5 durch einen vom Führungsspieß 2 durchbohrten Stopfen I6 abgedichtet, der durch eine durchbohrte Schraubkappe I7 auf dem Ende I4 gehalten wird.

Da der Flüssigkeitskanal I5 bei guter Führung des Schneidinstruments I durch den Führungsspieß 2 unter Umständen keinen ausreichend großen, ringförmigen Querschnitt hat, weist der Querschnitt 18 des Führungsspießes 2 an seinem Umfang 19 einen Umfangsbereich 20 auf, der von der ansonsten vorhandenen Kreisform des Umfangs 19 erfolgt, im Sinne einer Verminderung des Querschnitts 18 gegenüber kreisförmiger Ausbildung. Auf diese Weise ist am Führungsspieß 2 in Längsrichtung eine Abflachung, nämlich am Umfangsbereich 20 vorgesehen, durch welche der Flüssigkeitskanal 15 erweitert ist und die Passage größerer Kühlmittelmengen zuläßt.

In der Zeichnung oberhalb der Queröffnung 13 ist die Abflachung am Umfangsbereich 20 nicht vorgesehen und auch unnötig. Auf diese Weise ist die einwandfreie Abdichtung durch den Stopfen 16 gewährleistet. Der Querschnitt 18 stellt also einen Schnitt durch den Führungsspieß von der Queröffnung 13 abwärts dar.

Der durchbohrte Stopfen 16 kann durch einen undurchbohrten Stopfen 21 ersetzt werden, wenn das Schneidinstrument 1 ohne Führungsspieß 2 verwendet werden soll.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Rotierendes Schneidinstrument für chirurgische Zwecke mit Führungsspieß, für den im Schneidinstrument ein Längskanal vorgesehen ist,

**dadurch gekennzeichnet,**

daß eine das Schneidinstrument (1) umgreifende, abgedichtete und drehbar auf dem Schneidinstrument gelagerte Muffe (7) zur Kühlmittelzufuhr und eine zugeordnete Queröffnung (13) im Schneidinstrument vorgesehen ist und daß sich zwischen dem Führungsspieß (2) und der Wand des Längskanals (3) ein Flüssigkeitskanal (15) befindet, der an dem Ende (14) des Schneidinstruments abgedichtet ist, das dessen Schneidende (6) gegenüberliegt.

2. Schneidinstrument nach Anspruch 1, **dadurch gekennzeichnet,** daß der Querschnitt (18) des Führungsspießes (2) einen fast ebenso großen Durchmesser hat wie der Innendurchmesser des kreiszylindrischen Längskanals (3), jedoch von der Kreisform abweichende Umfangsbereiche (20) aufweist zur Bildung des Flüssigkeitskanals (15).

3. Schneidinstrument nach Anspruch 1 oder 2, **dadurch gekennzeichnet,** daß zur Führung der Muffe (7) wenigstens ein Wälzlager (9) vorgesehen ist.

4. Schneidinstrument nach einem der vorangehenden Ansprüche, **dadurch gekennzeich-**

net, daß zur Abdichtung der Muffe (7) und/oder des Flüssigkeitskanals (15) wenigstend ein Dichtungsring (10) oder durchbohrter Stopfen (16) an der Muffe (7) bzw. an dem dem Schneidende (6) gegenüberliegenden Ende (14) vorgesehen ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE-A-1 752 125 (CLEVELAND TWIST DRILL CO.)<br>* Seite 4, Zeile 16 - Seite 5, Zeile 12; Figuren 1,2 * | 1 | A 61 B 17/16 |
| A,D | US-A-4 021 920 (H. KIRSCHNER)<br>* Figuren 1-4; Patentanspruch 1 *<br>& DE-A-2 331 023 | 1 | |
| A,D | MECRON KATALOG, 1979/80, B. OSTEOSYNTHESE, Seite B.33, Katalog-Nrn. 836 und 839 | | |

|  | RECHERCHIERTE SACHGEBIETE (Int. Cl.4) |
|---|---|
|  | A 61 B<br>A 61 C<br>B 23 B<br>B 23 Q<br>F 16 L |

Der vorliegende Recherchenbericht wurde für alle Patentansprüche erstellt.

| Recherchenort | Abschlußdatum der Recherche | Prüfer |
|---|---|---|
| DEN HAAG | 19-06-1987 | NEILL M.C. |